# EUROPÄISCHE PATENTSCHRIFT

(11) **EP 0 276 008 B1**
(45) Veröffentlichungstag und Bekanntmachung des Hinweises auf die Patenterteilung: **20.10.1993**
(21) Anmeldenummer: 88100877.5
(22) Anmeldetag: 21.01.1988
(51) Int. Cl.: C12P 21/08, G01N 33/68, G01N 33/86, C12N 5/18

(54) **Fibrinspezifischer Antikörper, Verfahren zu seiner Herstellung und seine Verwendung**
Fibrin-specific antibody, method for its production and its use
Anticorps fibrine-spécifique, procédé pour sa production et son utilisation

(30) Priorität: 22.01.1987 DE 3701812
(43) Veröffentlichungstag der Anmeldung: 27.07.1988
(73) Patentinhaber: Max-Planck-Gesellschaft zur Förderung der Wissenschaften e.V., D-37073 Göttingen (DE)
(72) Erfinder: Müller-Berghaus, Gert, Prof. Dr., D-6300 Giessen-Allendorf (DE); Scheefers-Borchel, Ursula, Dr., D-6307 Linden-Grossenlinden (DE)
(74) Vertreter: Huber, Bernhard, Dipl.-Chem.

(56) Entgegenhaltungen:
- EP-A- 0 152 612
- CHEMICAL ABSTRACTS, Band 100, 1984, Seite 433, Nr. 49769e, Columbus, Ohio, US; K.Y. HUI et al.: "Monoclonal antibodies to a synthetic fibrin-like peptide bind to human ibrin but not fibrinogen"
- CHEMICAL ABSTRACTS, Band 100, 1984, Seite 427, Nr. 119083c, Columbus, Ohio, US; B. KUDRYK et al.: "Specificity of a monoclonal antibody for the amino-terminal region of fibrin"
- CHEMICAL ABSTRACTS, Band 109, 1988, Seite 761, Nr. 190847w, Columbus, Ohio, US; G.R. MATSUEDA et al.: "Antibodies that bind to a protein but not its precursor. Synthesis of fibrin-unique peptides and selection of high-affinity, fibrin-specific antibodies"
- TROMBOSIS AND HAEMOSTASIS, Band 58, Nr. 1, 1987, Seite 266, DE; U. SCHEEFERS-BORCHEL et al.: "A new fibrin-specific antibody discriminating between fibrin and fibrinogen is directed against the synthetic peptide Leu-Ile-Asp-Gly-Lys-Met"
- AMERICAN HEART ASSOCIATION MONOGRAPH, Band 0, Nr. 114, 1985, Seiten III-192, US; R.K. ITO et al.: "Development of a thrombolytic agent using a human fibrin-specific monoclonal antibody as a conjugate-carrier for streptokinase"

## Beschreibung

Die Erfindung betrifft einen mit der Peptidsequenz Leu-Ile-Asp-Gly-Lys-Met spezifisch bindefähigen und Fibrin und Fibrinogen diskriminierenden Antikörper, ein Verfahren zu seiner Herstellung, seine Verwendung und ein ihn enthaltendes Reagenz.

Fibrin entsteht im Laufe des Gerinnungsprozesses aus Fibrinogen. Proteolytische Enzyme wie z. B. Thrombin können sowohl intra- als auch extravasal Peptide aus dem löslichen Plasmaprotein Fibrinogen abspalten. Dabei entsteht Fibrinmonomer. Mehrere Fibrinmonomere können sich zu einem Fibrinpolymer, einem Fibringerinsel zusammenlagern. Wenn dies intravasal geschieht, so kann dies zu deletären Folgen führen. Bei Patienten mit disseminierter intravasaler Gerinnung, die auch als Verbrauchskoagulopathie bezeichnet wird, tritt lösliches Fibrin im zirkulierenden Blut auf. Es kann sich in Kapillaren und kleinen Blutgefäßen zu Fibrinsträngen zusammenlagern, die licht- und elektronenmikroskopisch nachweisbar sind (Übersicht bei Müller-Berghaus, G., Seminars in Thrombosis and Hemostasis 3: 209-246, 1977). Auch bei entzündlichen Erkrankungen kann im Gewebe intra- oder extravaskulär ein Material nachgewiesen werden, das sich von Fibrinogen ableitet.

Es wäre nun wünschenswert, Fibrin in Blut, Plasma oder Gewebe spezifisch nachweisen zu können. Bis vor kurzer Zeit gab es nur qualitative Methoden zum Nachweis von Fibrin, da Fibrin sich von Fibrinogen ableitet und sich nur durch das Fehlen der Fibrinopeptide unterscheidet. Die qualitativen bzw. semiquantitativen Methoden beruhen z. B. auf einer Fällung nach Zugabe von Ethanol (Godal, H.C., Abildgaard, U., Scand. J. Haematol. 3: 342-350, 1966), Protaminsulfat (Niewiarowski, S., Gurewich, V., J. Lab. Clin. Med. 77: 665-676, 1971) oder Agglutination von beschichteten Erythrozyten oder Latexpartikeln (Largo, R., Heller, V., Straub, P. W., Blood 47: 991-1002, 1976). Diese zitierten Methoden erlauben nicht zwischen Fibrin und Fibrinogen zu unterscheiden. Andere Methoden versuchen Fibrin vom Fibrinogen durch Chromatographie zu trennen (Alkjaersig, N., Fletcher, A., Burstein, R., Am. J. Obstet. Gynecol. 122: 199-209, 1975; Heene, D. L., Matthias, F. R., Thromb. Res. 2: 137-154, 1973). Auch diese Methoden sind nicht spezifisch, da Fibrin sich nicht völlig von Fibrinogen abtrennen läßt. Fibrinogen ist notwendig, um Fibrin in Lösung zu halten (Krell, W., Mahn, I., Müller-Berghaus, G., Thromb. Res. 14: 299-310, 1979).

Eine weitere Methode, um im gereinigten System Fibrin von Fibrinogen zu unterscheiden, ist die Bestimmung des N-terminalen Glycins (Kierulf, P., Godal, H. C., Scand. J. Haematol. 9: 370-372, 1972). Diese Methode ist jedoch kompliziert und benötigt große Mengen an Ausgangsmaterial, so daß sie sich als Test nicht durchgesetzt hat.

Aus EP 0 152 612 ist nun ein Verfahren zur qualitativen und quantitativen Bestimmung von Fibrin bekannt, bei dem ein monoklonaler Antikörper verwendet wird, der spezifisch mit Fibrin, nicht jedoch mit Fibrinogen reagiert. Dieser Antikörper wird erhalten, indem man Peptide als Immunogene verwendet, die der Aminosäuresequenz der terminalen Gruppen der alpha-Kette von Fibrin entsprechen. Mit diesem Antikörper läßt sich jedoch kein genügend genaues Nachweisverfahren nach dem Prinzip des Immunoassays durchführen, da hierfür zwei verschiedene, spezifisch mit Fibrin bindefähige Antikörper notwendig sind.

Bestimmungsverfahren nach dem Prinzip des Immunoassays sind weit verbreitet. Vorteil dieser Bestimmungsmethode ist ihre Genauigkeit und die Möglichkeit, sehr geringe Substanzmengen nachweisen zu können. Zur Durchführung der Bestimmung sind verschiedene Verfahrensvarianten möglich sowohl mit homogenen als auch mit heterogenen Phasen. Bei der Ausführungsform mit heterogener Phase wird einer der Rezeptoren an einen Träger gebunden. Beim Sandwich-Verfahren wird beispielsweise ein Rezeptor an den Träger gebunden, die Testlösung zugegeben, wobei das in der Testlösung enthaltene, zu bestimmende Antigen an den Rezeptor gebunden wird. Dann wird ein markierter Rezeptor zugegeben, der spezifisch mit dem Antigen oder dem Antigen-Antikörperkomplex reagiert. Über den markierten Antikörper kann dann die Menge an Antigen berechnet werden. Für dieses allgemeine Prinzip gibt es viele Variationsmöglichkeiten. So kann beispielsweise eine Bestimmung mit drei Rezeptoren erfolgen, wobei einer der drei Rezeptoren in heterogener Phase vorliegt und die anderen beiden Rezeptoren löslich sind und wobei einer der beiden löslichen Rezeptoren markiert ist, während der andere unmarkiert ist. Der unlösliche Rezeptor ist dann gegen den nicht markierten löslichen Rezeptor gerichtet.

Es war nun Aufgabe der vorliegenden Erfindung, ein Verfahren zur qualitativen und quantitativen spezifischen Bestimmung von Fibrin zur Verfügung zu stellen, das insbesondere den Nachweis sehr geringer Mengen an Fibrin ermöglicht, das sehr genau durchzuführen ist und das insbesondere den spezifischen Nachweis des Fibrins ermöglicht, ohne daß ebenfalls vorhandenes Fibrinogen oder andere im Blut, Plasma oder Gewebe des menschlichen Organismus vorhandene Proteine stören.

Diese Aufgabe wird gelöst durch einen fibrin- spezifischen Antikörper, der spezifisch bindefähig ist mit der Peptidsequenz Leu-Ile-Asp-Gly-Lys-Met und Fibrin und Fibrinogen diskriminiert.

Antiseren, die die erfindungsgemäß verwendeten Antikörper enthalten, werden erhalten, indem man in den Organismus von Versuchstieren als Immunogen Peptide, die die Sequenz Leu-lle-Asp-Gly-Lys-Met enthalten, einbringt. Bevorzugt werden Peptide mit 6 bis 20 Aminosäuren, die diese Sequenz enthalten, verwendet. Als vorteilhaft hat sich ein Hexapeptid der Formel Leu-Ile-Asp-Gly-Lys-Met erwiesen. Dieses wird in üblicher Weise, zweckmäßig unter Einschaltung einer "spacer"-Gruppierung, an hierfür geeignete Trägerpeptide gebunden.

Die obengenannten Oligopeptide werden in bekannter Weise synthetisiert. Geeignete Verfahren sind beispielsweise in E. Wünsch in Houben-Weyl, "Methoden der organischen Chemie", Band XV/1 und 2; The Peptides, Vol. 1, "Major Methods of Peptide Bond Formation", Academic Press (1979), oder "Perspectives in Peptide Chemistry", Karger, Basel (1981), beschrieben.

Dabei werden Aminosäuren oder Peptidfragmente, deren funktionelle Gruppen, die nicht an der Reaktion teilnehmen sollen, in geeigneter Weise geschützt sind, zur Reaktion gebracht. Die freien Carboxygruppen von Aminosäurederivaten oder Peptidfragmenten werden in geeigneter Weise aktiviert und mit der Aminogruppe von Aminosäurederivaten oder teilgeschützten Peptidfragmenten umgesetzt. Die Aktivierung kann mit DCCI, DCCI/HOBt, DCCI/Hydroxysuccinimid, POCb, Chlorameisensäureestern oder der Azid-Methode erfolgen. Bevorzugt verwendet man die DCCI/HOBt-Methode nach Chem. Ber. 103, 788 (1970).

Der Aufbau der teilgeschützten Oligopeptide kann stufenweise vom C-terminalen Ende her oder auch durch Fragmentkopplungen erfolgen. Auch ein stufenweiser Aufbau an einem löslichen oder unlöslichen polymeren Träger, wie beispielsweise Polyoxyethylen oder vernetztes Polystyrol, ist möglich. Als Schutzgruppe für die alpha-Aminofunktion der Aminosäuren oder Peptidfragmente kommen t-Butyloxycarbonyl (Boc), Fluorenylmethoxycarbonyl (Fmoc), Benzyloxycarbonyl (Z), alpha,alpha-Dimethyl-3,5-dimethoxybenzyloxycarbonyl, 2-(Biphenylyl-(4)-propyl-(2)-oxycarbonyl und Trityl in Frage. Bevorzugt verwendet man Boc, Fmoc und Z.

Als Schutz für die alpha-Carboxygruppe von Aminosäuren und Peptidfragmenten verwendet man die Veresterung mit Alkanolen oder araliphatischen Alkoholen. Bevorzugt sind die Methyl-, Benzyl- und tert. Butylester.

Der N-Terminus der verwendeten Oligopeptide muß zur eindeutigen Reaktionsführung bei der Kopplung an das Trägerprotein so geschützt sein, daß eine Abspaltung der Schutzgruppe am Peptid-Protein-Konjugat ohne Schädigung des Proteins erfolgen kann. Dazu eignen sich die Fmoc-Schutzgruppe, die unter alkalischen Bedingungen sowie die 2-(Biphenyl-(4)-propyl-(2)-oxycarbonyl-, alpha,alpha-Dimethyl-3,5-dimethoxybenzyloxycarbonyl-, Boc- und Tritylgruppe, die unter relativ milden sauren Bedingungen abgespalten werden können.

Als "spacer"-Gruppierung kommen die in der Peptidchemie üblicherweise verwendeten Brückenglieder in Betracht, wie sie beispielsweise auch bei der Immobilisierung von Enzymen verwendet werden. Die entsprechenden Reagenzien enthalten zwei gleiche oder verschiedene reaktive Gruppen an den Enden einer Alkylengruppe mit beispielsweise 2 bis 8 Kohlenstoffatomen, wobei als reaktive Gruppen solche in Betracht kommen, die mit den funktionellen Gruppen der Aminosäuren eine kovalente Bindung eingehen können. Geeignet sind beispielsweise Dialdehyde wie Glutaraldehyd, Diisocyanate wie 1,6-Hexamethylendiisocyanat, Diamine wie 1,6-Hexamethylendiamin oder _{M}-Aminocarbonsäuren wie _{E}-Aminocapronsäuren. Diese bifunktionellen Reagenzien werden - gegebenenfalls nach Aktivierung - in bekannter Weise einerseits mit dem Oligopeptid und andererseits mit dem Träger verknüpft. Als Träger kommen grundsätzlich beliebige Proteine in Betracht. Zweckmäßig werden solche Trägerproteine ausgewählt, die keine Kreuzreaktion verursachen. Geeignet sind beispielsweise Albumine wie Rinderserum-Albumin oder Haemocyanine wie Keyhole limpet hemocyanine.

Mit den so erhaltenen Antigenen werden Versuchstiere wie Mäuse, Ratten, Kaninchen oder Ziegen in bekannter Weise immunisiert und so Antiseren mit polyklonalen Antikörpern erhalten. In einer bevorzugten Ausgestaltung werden in entsprechender Weise monoklonale Antikörper nach der Methode von G. Köhler und C. Milstein, Nature 256, 495-497, (1975) erzeugt.

Daher ist weiterhin Gegenstand der Erfindung ein Verfahren zur Gewinnung eines monoklonalen Antikörpers, der spezifisch mit der Peptidsequenz Leu-Ile-Asp-Gly-Lys-Met bindefähig ist, das dadurch gekennzeichnet ist, daß man Mäuse mit einem Peptid, das die Aminosäuresequenz Leu- Ile-Asp-Gly-Lys-Met trägt immunisiert, B-Lymphozyten aus der Milz der immunisierten Tiere mit Myelomzellen fusioniert, die gebildeten Hybridomazellen kloniert, die mit der Sequenz Leu-Ile-Asp-Gly-Lys-Met bindefähigen Klone isoliert und züchtet und den durch sie gebildeten monoklonalen Antikörper gewinnt.

Besonders bevorzugt wird eine Zellinie verwendet, die selbst kein Immunglobulin produziert.

Die erfindungsgemäß erhältlichen monoklonalen Antikörper reagieren nicht mit Fibrinogen, wohl aber mit desAA-Fibrin (Fibrin Typ I), desAABB-Fibrin (Fibrin Typ 11), dem Peptidkonjugat und dem synthetischen Peptid. Weiterhin reagieren die monoklonalen Antikörper nicht mit Plasmininduzierten Spaltprodukten des Fibrinogens oder Fibrins.

Ein weiterer Gegenstand der Erfindung ist die Verwendung des erfindungsgemäßen fibrinspezifischen Antikörpers zur qualitativen und/oder quantitativen Bestimmung von Fibrin.

Überraschenderweise wurde gefunden, daß bei Verwendung des erfindungsgemäßen fibrinspezifischen Antikörpers, der spezifisch mit der Sequenz Leu-Ile-Asp-Gly-Lys-Met bindefähig ist, Fibrin spezifisch gebunden wird, während Fibrinogen nicht gebunden wird. Es ist somit möglich, unter Verwendung dieses Antikörpers Fibrin spezifisch in Körperflüssigkeiten oder Körpergeweben nachzuweisen.

Die Verwendung des erfindungsgemäßen fibrinspezifischen Antikörpers zur selektiven quantitativen Bestimmung von Fibrin nach dem Prinzip des Immunoassays durch Inkubation mit mindestens zwei verschiedenen Rezeptoren, von denen der erste Rezeptor R₁ in fester Phase vorliegt und mit dem Fibrin oder einem Komplex aus Fibrin und einem Rezeptor bindefähig ist und mindestens ein weiterer Rezeptor R₂, der mit Fibrin oder einem Komplex aus Fibrin und einem Rezeptor bindefähig ist, in flüssiger Phase gelöst vorliegt und wobei ein Rezeptor R₂ eine Markierung trägt, Trennung der festen von der flüssigen Phase und Messung der Markierung in einer der Phasen, ist dadurch gekennzeichnet, daß man als einen der Rezeptoren einen fibrinspezifischen Antikörper verwendet, der spezifisch mit der Peptidsequenz Leu-Ile-Asp-Gly-Lys-Met bindefähig ist und zwischen Fibrin und Fibrinogen diskriminiert.

Zur Durchführung dieses Verfahrens werden mindestens zwei verschiedene Rezeptoren verwendet. Einer von diesen Rezeptoren ist spezifisch mit der Peptidsequenz Leu-Ile-Asp-Gly-Lys-Met bindefähig. Mindestens ein weiterer Antikörper muß ebenfalls mit Fibrin oder einem Komplex aus Fibrin und einem Rezeptor bindefähig sein. Hier kann ein Antikörper verwendet werden, der Fibrin unspezifisch bindet und auch mit Fibrinogen eine Bindung eingeht. Bevorzugt wird jedoch auch hier ein Rezeptor verwendet, der spezifisch bindefähig mit Fibrin ist, wie beispielsweise der aus EP-A 0152 612 bekannte.

Einer der beiden Rezeptoren wird an eine Festphase gebunden. Die Bindung an die Festphase erfolgt in an sich bekannter Weise und ist dem Fachmann bekannt. Weiterhin wird mindestens ein weiterer Rezeptor verwendet, der in löslicher Form vorliegt. Dieser weitere Rezeptor trägt eine Markierung. Werden mehrere weitere Rezeptoren R₂ verwendet, so trägt nur einer von diesen eine Markierung. Die Markierung des Rezeptors erfolgt in an sich üblicher Weise und ist dem Fachmann bekannt.

Die Markierung erfolgt in an sich bekannter Weise bevorzugt durch eine radioaktive Verbindung, ein Enzym, eine chemilumineszente oder fluoreszierende Verbindung. Besonders bevorzugt wird die Markierung mit einem Enzym vorgenommen, insbesondere mit Peroxydase oder Phosphatase.

In einer besonders bevorzugten Ausführungsform des Verfahrens wird nun entweder ein unspezifisch mit Fibrin bindefähiger Rezeptor oder bevorzugt ein spezifisch mit Fibrin bindefähiger Rezeptor an eine Festphase gebunden. Dieser an die Festphase gebundene Rezeptor wird anschließend mit der das zu bestimmende Fibrin enthaltenden Lösung und einem Antikörper, der spezifisch mit der Peptidsequenz Leu-Ile-Asp-Gly-Lys- Met bindefähig ist, in löslicher Form vorliegt und eine Markierung trägt, inkubiert. Ist der an die Festphase gebundene Rezeptor ein unspezifisch mit Fibrin bindefähiger, so lagern sich an der Festphase nicht nur Fibrin- sondern auch Fibrinogenmoleküle an. Der zweite Antikörper, der spezifisch mit der Peptidsequenz Leu-Ile-Asp-Gly-Lys-Met bindefähig ist, lagert sich dann nur an das Fibrin an, so daß nur die Fibrinmoleküle spezifisch einen markierten Antikörper tragen, während die Fibrinogenmoleküle nicht markiert werden. Auf diese Weise ist es nach Trennung der festen von der flüssigen Phase möglich, über die Messung der Markierung den Gehalt an Fibrin zu bestimmen.

Wird als an die Festphase gebundener Rezeptor ein mit Fibrin spezifisch bindefähiger Rezeptor verwendet, z. B. der aus EP-A 0152 612 bekannte, so wird an der Festphase spezifisch nur Fibrin gebunden und bei der Inkubation mit dem löslichen Antikörper reagiert auch dieses wiederum spezifisch mit dem Fibrin. Da somit praktisch keine Bindung des Fibrinogens an die Festphase erfolgt, ist dieses Verfahren noch spezifischer und erlaubt daher sehr genaue Bestimmungen.

In einer weiteren besonders bevorzugten Ausführungsform des erfindungsgemäßen Verfahrens wird ein gegen die Peptidsequenz Leu-Ile-Asp-Gly-Lys-Met spezifisch bindefähiger Antikörper an einem Träger immobilisiert. Mit diesem immobilisierten Antikörper wird dann die das zu bestimmende Fibrin enthaltende Lösung und ein mit Fibrin bindefähiger Rezeptor, vorzugsweise ein spezifisch mit Fibrin bindefähiger Rezeptor inkubiert. Dabei wird an der Festphase praktisch ausschließlich Fibrin gebunden, Fibrinogen bleibt in Lösung. Weiterhin lagert sich an das Fibrin der lösliche, markierte, mit Fibrin bindefähige Antikörper an. Nach Trennung der festen von der flüssigen Phase kann wiederum über die Markierung der Gehalt an Fibrin sehr genau bestimmt werden.

Weitere, dem Fachmann bekannte Verfahrensvarianten mit drei Rezeptoren sind ebenfalls unter Verwendung des spezifisch mit der Peptidsequenz Leu-Ile-Asp-Gly-Lys-Met spezifisch bindefähigen Antikörpers möglich und bedürfen hier keiner weiteren Ausführungen.

Von den für die Durchführung des erfindungsgemäßen Verfahrens verwendeten Antikörpern ist bevorzugt mindestens einer ein monoklonaler Antikörper. Besonders bevorzugt werden als Rezeptoren nur monoklonalen Antikörper verwendet.

Ein weiterer Gegenstand der Erfindung ist ein Reagenz zur selektiven Bestimmung von Fibrin, enthaltend einen an eine Festphase gebundenen Rezeptor Ri, der mit Fibrin oder einem Komplex aus Fibrin und einem Rezeptor bindefähig ist und mindestens einen in löslicher Phase vorliegenden Rezeptor R₂, der mit Fibrin oder einem Komplex aus Fibrin und einem Rezeptor bindefähig ist, wobei ein löslicher Rezeptor R₂ eine Markierung trägt, das dadurch gekennzeichnet ist, daß einer der Rezeptoren ein Antikörper ist, der spezifisch mit der Peptidsequenz Leu-Ile-Asp-Gly-Lys-Met bindefähig ist.

Der mit der Peptidsequenz Leu-Ile-Asp-Gly-Lys-Met spezifisch bindefähige Antikörper kann entweder an die Festphase gebunden vorliegen oder aber als löslicher markierter oder unmarkierter Rezeptor verwendet werden. Bevorzugt ist dieser Antikörper ein monoklonaler Antikörper. Besonders bevorzugt sind alle verwendeten Rezeptoren monoklonale Antikörper.

Weiterhin kann der erfindungsgemäße fibrin- spezifische Antikörper vorteilhaft zur qualitativen Bestimmung von Fibrin und Fibringerinnseln im menschlichen Blutkreislauf verwendet werden.

Dazu wird bevorzugt der erfindungsgemäße fibrinspezifische Antikörper, besonders bevorzugt der erfindungsgemäß hergestellte monoklonale fibrinspezifische Antikörper markiert, in den Blutkreislauf injiziert und durch geeignete Mittel sichtbar gemacht.

Besonders bevorzugt wird Patienten der erfindungsgemäße fibrinspezifische Antikörper, der radioaktiv markiert wurde, injiziert. Der Antikörper reichert sich an den Thromben an und kann mit einer gamma-Kamera nachgewiesen werden. Auf diese Weise kann nicht nur die Lage des Thrombus, sondern auch seine Größe ermittelt werden.

Weiterhin kann der fibrinspezifische Antikörper dazu benutzt werden, Medikamente herzustellen, die ein Fibringerinnsel, z. B. einen Thrombus lysieren können, gezielt zu diesem Gerinnsel zu führen, indem man die entsprechenden Medikamente an die Antikörper koppelt. Als lysierende Substanzen können beispielsweise Gewebe-Plasminogen-Aktivator, Urokinase oder Streptokinase verwendet werden.

Da der erfindungsgemäß verwendete Antikörper nicht mit Fibrinogen reagiert, führt diese Therapie nicht zu einer nennenswerten Reduktion des Plasmafibrinogens, die immer Folge einer herkömmlichen Lysetherapie ist.

Erfindungsgemäß wird ein Antikörper zur Verfügung gestellt, der spezifisch mit Fibrin reagiert und Fibrinogen nicht bindet. Mit diesem Antikörper ist es möglich, Fibrin im Blut spezifisch sowohl qualitativ als auch quantitativ nachzuweisen. Der erfindungsgemäße Antikörper erlaubt es, genaue Aussagen über den Fibringehalt des Blutes zu machen und andererseits Thromben, die sich in Blutgefäßen gebildet haben und zu Gefäßverschlüssen führen können, zu ermitteln. Weiterhin ist es möglich, gebildete Thromben spezifisch aufzulösen.

Die Erfindung wird noch durch die Zeichnung und die folgenden Beispiele erläutert.

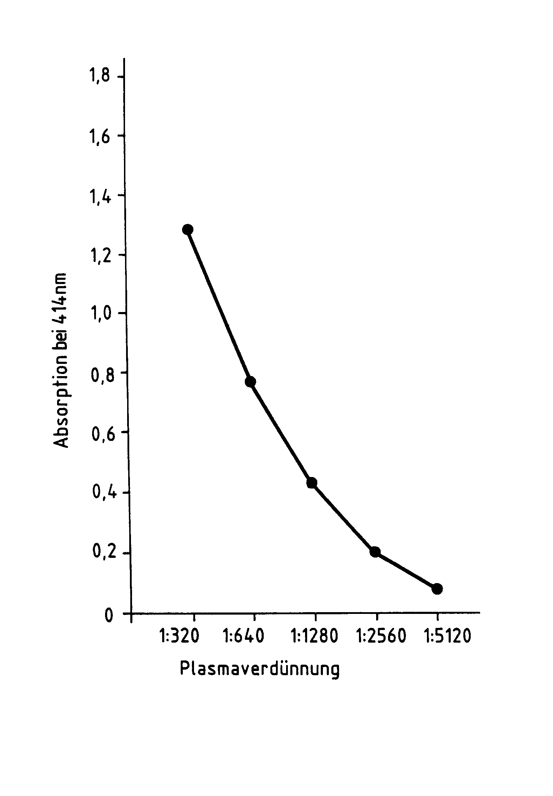

Fig. 1 zeigt in einem Diagramm Ergebnisse, die bei der Bestimmung von löslichem Fibrin in Plasma, das durch Zugabe von Thrombin erzeugt wurde, erhalten wurden. Kontrollplasma, dem kein Thrombin zugesetzt wurde, zeigte bei einer Verdünnung von 1:20 eine Absorption bei 414 nm von 0,094.

In den folgenden Beispielen wurden die nachstehenden Abkürzungen verwendet:
Asp
L-Asparaginsäure
Boc
t-Butyloxycarbonyl
DCCI
Dicyclohexylcarbodiimid
desAA-Fibrin
Typ I
desAABB-Fibrin
Typ 11
ELISA
Enzymimmunoassay (enzyme linked immunosorbent assay)
Fmoc
Fluorenylmethoxycarbonyl
Gly
Glycin
HOBt
1-Hydroxy-1 H-benzotriazol
Ile
L-Isoleucin
i^{.} p^{.}
intraperitoneal
i. v.
intravenös
KLH
Keyhole limpet hemocyanine (Haemocyanin einer marinen Napfschnecke)
Leu
L-Leucin
Lys
L-Lysin
Met
L-Methionin
PBS
Phosphatgepufferte Kochsalzlösung (phosphate buffered saline)
RSA
Rinderserum-Albumin
s. c.
subcutan
Z
Benzyloxycarbonyl

### Herstellung monoklonaler Antikörper

### Beispiel 1

Ein an KLH als Trägerprotein gekoppeltes Peptid mit der Sequenz Leu-Ile-Asp-Gly-Lys-Met wird in PBS gelöst und zu gleichen Teilen mit Freund-Adjuvans gemischt. Jeweils 100 ug dieses Gemisches werden in 6 bis 8 Wochen alte Balb/c Mäuse i.p. und s.c. injiziert. Diese Injektionen werden zweimal im Abstand von 3 bis 4 Wochen wiederholt. Nach obengenanntem Schema mit konjugiertem Peptid (RSA, KLH) immunisierte Mäuse erhalten 3 Tage vor Entnahme der Milz eine i.v. Injektion von 100 ug konjugiertem Peptid, welches in PBS gelöst wird.

Etwa 10⁸ Zellen von der Milz einer immunisierten Maus werden mit 5 x 10⁷ Myelomazellen (x63-Sp8-653, eine Linie, die kein Immunglobulin synthetisiert; zu beziehen bei The Salk Institute, Cell Cistribution Center, San Diego CA 92112, USA) in Gegenwart von Polyethylenglykol (MG 3400) fusioniert. Fusionierte Zellen werden auf 4 Platten, die jeweils 24 Vertiefungen enthalten, ausgesät. Jede dieser Vertiefungen enthält 5x10⁷ Milzzellen unimmunisierter syngener Mäuse in Nährmedium, welches Hypoxanthin, Aminopterin und Thymidin enthält.

Die Antikörper enthaltenden Überstände dieser fusionierten Zellen (Hybridoma) werden 10 bis 14 Tage später mittels ELISA bezüglich ihrer Spezifität gegen folgende Antigene getestet: Fibrinogen, desAA-Fibrin, desAABB-Fibrin und unkonjugiertes Peptid.

Um monoklonale Antikörper zu erhalten, die nur gegen Fibrin und gegen das synthetische Peptid gerichtet sind, werden Hybridomazellen, deren Überstände keine gegen Fibrinogen gerichteten Antikörper enthalten, zweimal kloniert.

Bestimmung von löslichem Fibrin in Plasma mit monoklonalen Fibrin-spezifischen Antikörpern

### Beispiel 2

Es wird lösliches Fibrin im Plasma mit einem ELISA bestimmt. Hierzu werden gemäß Beispiel 1 erhaltene monoklonale, Fibrin-spezifische Antikörper, die in PBS, pH 7,2, gelöst sind und die gegen das Peptid mit der Sequenz Leu-Ile-Asp-Gly-Lys-Met gerichtet sind, an Polystyrol als Träger immobilisiert. Nach einem Waschschritt wird verdünntes Plasma, welches lösliches Fibrin enthält, dazugegeben. Das Plasma wird in einem Puffer der PBS 5 mmol EDTA, 2mmol EACA 200 E Aprotinin/ ml und 0,2 % Tween 20 enthält, verdünnt. Dieses lösliche Fibrin wurde durch Zugabe geringer Mengen von Thrombin zu Plasma hergestellt. Die Thrombinbehandlung wurde so gewählt, daß das Plasma keine Trübung und keine Fibrinfäden zeigte, das heißt, das entstandene Fibrin lag als lösliches Fibrin vor. Die Thrombinbehandlung wurde durch Zugabe von Hirudin (5 Einheiten/ml Plasma) gestoppt. Nach einem Waschschritt im PBS, 0,1 % Tween 20 wird das an den Antikörper gebundene Fibrin mit einem polyklonalen Antikörper, der sowohl Fibrin als auch Fibrinogen bindet und an den Phosphatase gekoppelt ist, der gelöst ist in PBS, das 0,2 % Tween 20 enthält und einen pH von 7,2 hat, eine Stunde bei Raumtemperatur inkubiert. Nach einem erneuten Waschschritt wird durch Zugabe von p-Nitrophenylphosphat-di-Na-hexahydrat eine Änderung der optischen Dichte in den Reaktionsgefäßen gemessen, in denen die monoklonalen Antikörper mit dem löslichen Fibrin reagiert haben. Die Ergebnisse sind der Fig. 1 zu entnehmen.

Bestimmung von löslichem Fibrin in Plasma mit zwei verschiedenen Fibrin-spezifischen Antikörpern

### Beispiel 3

Der gemäß EP-A-0152612 erhältliche Fibrin- spezifische Antikörper, der gegen das aminoterminale Ende der alpha-Kette von Fibrin gerichtet ist, wird, wie im Beispiel 2 beschrieben, an einem Träger fixiert. Nach einem Waschschritt wird Plasma, das lösliches Fibrin enthält, mit dem monoklonalen Antikörper unter denselben Bedingungen wie im Beispiel 2 inkubiert. Nach einem weiteren Waschschritt wird das lösliche Fibrin durch einen zweiten erfindungsgemäßen Fibrin-spezifischen monoklonalen Antikörper, der gegen die Sequenz Leu-Ile-Asp-Gly-Lys-Met gerichtet ist, detektiert. Dieser zweite Fibrin-spezifische Antikörper trägt Peroxydase kovalent gebunden. Nach einem Waschschritt wird nach Zugabe von ABTS als Substrat für Peroxydase eine Änderung der optischen Dichte gemessen.

### Beispiel 4

Es wurde wie in Beispiel 3 beschrieben verfahren, jedoch wurde an den zweiten Fibrin-spezifischen Antikörper statt eines Enzyms Biotin gekoppelt. Vor Substratzugabe wurde dann Peroxidasekonjugiertes Avidin oder Peroxydase-konjugiertes Streptavidin zugesetzt. Die so gewählte Bestimmung von löslichem Fibrin erlaubt ganz spezifisch nur Fibrin zu bestimmen. Fibrinogen, das mit Fibrin assoziiert ist, um dieses in Lösung zu halten, stört die erfindungsgemäße Bestimmung des Fibrins nicht.

### Beispiel 5

Ein gemäß Beispiel 1 erhaltener, fibrinspezifischer Antikörper, der gegen die Sequenz Leu-lle-Asp-Gly-Lys-Met gerichtet ist, wurde radioaktiv markiert. Dieser markierte Antikörper wurde zu einer Lösung von 1 % BSA in PBS zugegeben, die ein durch Thrombin hergestelltes Gerinnsel enthielt. Der Anteil der an das Gerinnsel gebundenen Antikörper wurde in einem Szintillationszähler bestimmt. Zum Vergleich wurden zu einer ein Gerinnsel enthaltenden Pufferlösung radioaktiv markierte Antikörper zugegeben, die nicht mit Fibrin oder Fibrinogen reagieren. Ein Vergleich der Ergebnisse der Szintillometrie zeigte, daß die erfindungsgemäßen Antikörper spezifisch an das Gerinnsel binden, während die Kontrollantikörper keine Bindung mit dem Gerinnsel eingingen.

## Patentansprüche (Patentansprüche für folgende(n) Vertragsstaat(en) : AT, BE, CH, DE, FR, GB, GR, IT, LI, LU, NL, SE)

1. Fibrinspezifischer Antikörper, spezifisch bindefähig mit der Peptidsequenz Leu-Ile-Asp-Gly-Lys-Met und diskriminierend zwischen Fibrin und Fibrinogen.

2. Verfahren zur Gewinnung eines monoklonalen Antikörpers, der spezifisch mit der Peptidsequenz Leu-Ile-Asp-Gly-Lys-Met bindefähig ist und zwischen Fibrin und Fibrinogen diskriminiert, dadurch gekennzeichnet, daß man Mäuse mit einem Peptid, das die Aminosäuresequenz Leu-Ile-Asp-Gly-Lys-Met trägt immunisiert, B-Lymphocyten aus der Milz der immunisierten Tiere mit Myelomzellen fusioniert, die gebildeten Hybridomazellen kloniert, die mit der Sequenz Leu-Ile-Asp-Gly-Lys-Met bindefähigen Klone isoliert, auf Diskriminierung zwischen Fibrin und Fibrinogen selektioniert und züchtet und den durch sie gebildeten monoklonalen Antikörper gewinnt.

3. Verfahren nach Anspruch 2, dadurch gekennzeichnet, daß man Myelomzellen verwenden, die selbst kein Immunglobulin produzieren.

4. Verfahren nach Anspruch 2 oder 3, dadurch gekennzeichnet, daß man als Immunogen ein Konjugat verwendet, das aus einem Trägerpeptid besteht, an das über einen Spacer ein Peptid mit 6 bis 20 Aminosäuren gebunden ist, das die Sequenz Leu-Ile-Asp-Gly-Lys-Met enthält.

5. Verfahren nach Anspruch 4, dadurch gekennzeichnet, daß man als Immunogen ein Konjugat verwendet, bei dem an ein Trägerpeptid über einen Spacer ein Peptid mit der Sequenz Leu-Ile-Asp-Gly-Lys-Met gebunden ist.

6. Verfahren nach Anspruch 4 oder 5, dadurch gekennzeichnet, daß als Trägerpeptid ein Albumin oder ein Hämocyanin verwendet wird.

7. Verwendung des fibrinspezifischen Antikörpers von Anspruch 1 zur selektiven qualitativen und/oder quantitativen Bestimmung von Fibrin.

8. Verwendung des fibrinspezifischen Antikörpers nach Anspruch 1 zur selektiven Bestimmung von Fibrin nach dem Prinzip des Immunoassays durch Inkubation mit mindestens zwei verschiedenen Rezeptoren, von denen der erste Rezeptor R₁ in fester Phase vorliegt und mit dem Fibrin oder einem Komplex aus Fibrin und einem Rezeptor bindefähig ist und mindestens ein weiterer Rezeptor R₂, der mit Fibrin oder einem Komplex aus Fibrin und einem Rezeptor bindefähig ist, in flüssiger Phase gelöst vorliegt und wobei ein Rezeptor R₂ eine Markierung trägt, Trennung der festen von der flüssigen Phase und Messung der Markierung in einer der Phasen, dadurch gekennzeichnet, daß man als einen der Rezeptoren einen Antikörper verwendet, der spezifisch mit der Peptidsequenz Leu-Ile-Asp-Gly-Lys-Met bindefähig ist und zwischen Fibrin und Fibrinogen diskriminiert.

9. Verwendung nach Anspruch 8, dadurch gekennzeichnet, daß man als an die feste Phase gebundenen Antikörper einen Antikörper verwendet, der spezifisch mit der Peptidsequenz Leu-Ile-Asp-Gly-Lys-Met bindefähig ist und als löslichen Rezeptor einen anderen mit Fibrin bindefähigen Antikörper verwendet, der eine Markierung trägt.

10. Verwendung nach Anspruch 8, dadurch gekennzeichnet, daß man als an die Festphase gebundenen Rezeptor einen mit Fibrin bindefähigen Rezeptor verwendet und als löslichen Rezeptor einen Antikörper, der spezifisch mit der Peptidsequenz Leu-Ile-Asp-Gly- Lys-Met bindefähig ist und eine Markierung trägt, verwendet.

11. Verwendung nach einem der Ansprüche 8 bis 10, dadurch gekennzeichnet, daß die Markierung eine radioaktive Verbindung, ein Enzym, eine chemilumineszente oder fluoreszierende Verbindung ist.

12. Verwendung nach einem der Ansprüche 8 bis 11, dadurch gekennzeichnet, daß der Antikörper, der spezifisch mit der Peptidsequenz Leu-Ile-Asp-Gly-Lys-Met bindefähig ist, ein monoklonaler Antikörper ist.

13. Verwendung des fibrinspezifischen Antikörpers nach Anspruch 1 zur qualitativen und quantitativen Bestimmung in vitro von Fibrin und Fibringerinnseln im menschlichen Blutkreislauf und Gewebe.

14. Verwendung des fibrinspezifischen Antikörpers nach Anspruch 1 zur Ermittlung von Thromben in Blutgefäßen.

15. Fibrinspezifischer Antikörper nach Anspruch 1 zur Herstellung eines Arzneimittels zum spezifischen Auflösen von Thromben, dadurch gekennzeichnet, daß man an den fibrinspezifischen Antikörper eine lysierende Substanz koppelt.

16. Reagenz zur selektiven quantitativen Bestimmung von Fibrin, enthaltend einen an eine Festphase gebundenen Rezeptor Ri, der mit Fibrin oder einem Komplex aus Fibrin und einem Rezeptor bindefähig ist und mindestens einen in löslicher Phase vorliegenden Rezeptor R₂, der mit Fibrin oder einem Komplex aus Fibrin und einem Rezeptor bindefähig ist und wobei ein löslicher Rezeptor R₂ eine Markierung trägt, dadurch gekennzeichnet, daß einer der Rezeptoren ein Antikörper ist, der spezifisch mit der Peptidsequenz Leu-Ile-Asp-Gly-Lys-Met bindefähig ist und zwischen Fibrin und Fibrinogen diskriminiert.

## Patentansprüche (Patentansprüche für folgende(n) Vertragsstaat(en) : ES)

1. Verfahren zur Gewinnung eines monoklonalen Antikörpers, der spezifisch mit der Peptidsequenz Leu-Ile-Asp-Gly-Lys-Met bindefähig ist und zwischen Fibrin und Fibrinogen diskriminiert, dadurch gekennzeichnet, daß man Mäuse mit einem Peptid, das die Aminosäuresequenz Leu-Ile-Asp-Gly-Lys-Met trägt immunisiert, B-Lymphocyten aus der Milz der immunisierten Tiere mit Myelomzellen fusioniert, die gebildeten Hybridomazellen kloniert, die mit der Sequenz Leu-Ile-Asp-Gly-Lys-Met bindefähigen Klone isoliert, auf Diskriminierung zwischen Fibrin und Fibrinogen selektioniert und züchtet und den durch sie gebildeten monoklonalen Antikörper gewinnt.

2. Verfahren nach Anspruch 1, dadurch gekennzeichnet, daß man Myelomzellen verwenden, die selbst kein Immunglobulin produzieren.

3. Verfahren nach Anspruch 1 oder 2, dadurch gekennzeichnet, daß man als Immunogen ein Konjugat verwendet, das aus einem Trägerpeptid besteht, an das über einen Spacer ein Peptid mit 6 bis 20 Aminosäuren gebunden ist, das die Sequenz Leu-Ile-Asp-Gly-Lys-Met enthält.

4. Verfahren nach Anspruch 3, dadurch gekennzeichnet, daß man als Immunogen ein Konjugat verwendet, bei dem an ein Trägerpeptid über einen Spacer ein Peptid mit der Sequenz Leu-Ile-Asp-Gly-Lys-Met gebunden ist.

5. Verfahren nach Anspruch 3 oder 4, dadurch gekennzeichnet, daß als Trägerpeptid ein Albumin oder ein Hämocyanin verwendet wird.

6. Verwendung des nach Anspruch 1 hergestellten fibrinspezifischen Antikörpers zur selektiven qualitativen und/oder quantitativen Bestimmung von Fibrin.

7. Verwendung des nach Anspruch 1 hergestellten fibrinspezifischen Antikörpers zur selektiven Bestimmung von Fibrin nach dem Prinzip des Immunoassays durch Inkubation mit mindestens zwei verschiedenen Rezeptoren, von denen der erste Rezeptor R₁ in fester Phase vorliegt und mit dem Fibrin oder einem Komplex aus Fibrin und einem Rezeptor bindefähig ist und mindestens ein weiterer Rezeptor R₂, der mit Fibrin oder einem Komplex aus Fibrin und einem Rezeptor bindefähig ist, in flüssiger Phase gelöst vorliegt und wobei ein Rezeptor R₂ eine Markierung trägt, Trennung der festen von der flüssigen Phase und Messung der Markierung in einer der Phasen, dadurch gekennzeichnet, daß man als einen der Rezeptoren einen Antikörper verwendet, der spezifisch mit der Peptidsequenz Leu-Ile-Asp-Gly-Lys-Met bindefähig ist und zwischen Fibrin und Fibrinogen diskriminiert.

8. Verwendung nach Anspruch 7, dadurch gekennzeichnet, daß man als an die feste Phase gebundenen Antikörper einen Antikörper verwendet, der spezifisch mit der Peptidsequenz Leu-Ile-Asp-Gly-Lys-Met bindefähig ist und als löslichen Rezeptor einen anderen mit Fibrin bindefähigen Antikörper verwendet, der eine Markierung trägt.

9. Verwendung nach Anspruch 7, dadurch gekennzeichnet, daß man als an die Festphase gebundenen Rezeptor einen mit Fibrin bindefähigen Rezeptor verwendet und als löslichen Rezeptor einen Antikörper, der spezifisch mit der Peptidsequenz Leu-Ile-Asp-Gly- Lys-Met bindefähig ist und eine Markierung trägt, verwendet.

10. Verwendung nach einem der Ansprüche 7 bis 9, dadurch gekennzeichnet, daß die Markierung eine radioaktive Verbindung, ein Enzym, eine chemilumineszente oder fluoreszierende Verbindung ist.

11. Verwendung nach einem der Ansprüche 7 bis 10, dadurch gekennzeichnet, daß der Antikörper, der spezifisch mit der Peptidsequenz Leu-Ile-Asp-Gly-Lys-Met bindefähig ist, ein monoklonaler Antikörper ist.

12. Verwendung des nach Anspruch 1 hergestellten fibrinspezifischen Antikörpers zur qualitativen und quantitativen Bestimmung in vitro von Fibrin und Fibringerinnseln im menschlichen Blutkreislauf und Gewebe.

13. Verwendung des nach Anspruch 1 hergestellten fibrinspezifischen Antikörpers zur Ermittlung von Thromben in Blutgefäßen.

14. Reagenz zur selektiven quantitativen Bestimmung von Fibrin, enthaltend einen an eine Festphase gebundenen Rezeptor Ri, der mit Fibrin oder einem Komplex aus Fibrin und einem Rezeptor bindefähig ist und mindestens einen in löslicher Phase vorliegenden Rezeptor R₂, der mit Fibrin oder einem Komplex aus Fibrin und einem Rezeptor bindefähig ist und wobei ein löslicher Rezeptor R₂ eine Markierung trägt, dadurch gekennzeichnet, daß einer der Rezeptoren ein Antikörper ist, der spezifisch mit der Peptidsequenz Leu-Ile-Asp-Gly-Lys-Met bindefähig ist und zwischen Fibrin und Fibrinogen diskriminiert.

## Claims (Claims for the following Contracting State(s) : AT, BE, CH, DE, FR, GB, GR, IT, LI, LU, NL, SE)

1. Fibrin-specific antibody, specifically bindable with the peptide sequence Leu-Ile-Asp-Gly-Lys-Met and discriminating between fibrin and fibrinogen.

2. Process for the obtaining of a monoclonal antibody which is specifically bindable with the peptide sequence Leu-Ile-Asp-Gly-Lys-Met and discriminates between fibrin and fibrinogen, characterised in that one immunises mice with a peptide which carries the amino acid sequence Leu-Ile-Asp-Gly-Lys-Met, fusions B-lymphocytes from the spleen of the immunised animals with myeloma cells, clones the hybridoma cells formed, isolates the clones bindable with the sequence Leu-Ile-Asp-Gly-Lys-Met, selections for discrimination between fibrin and fibrinogen and cultures and recovers the monoclonal antibodies formed by them.

3. Process according to claim 2, characterised in that one uses myeloma cells which themselves produce no immune globulin.

4. Process according to claim 2 or 3, characterised in that, as immunogen, one uses a conjugate which consists of a carrier peptide to which is bound, via a spacer, a peptide with 6 to 20 amino acids and which contains the sequence Leu-Ile-Asp-Gly-Lys-Met.

5. Process according to claim 4, characterised in that, as immunogen, one uses a conjugate in which a peptide with the sequence Leu-Ile-Asp-Gly-Lys-Met is bound to a carrier peptide via a spacer.

6. Process according to claim 4 or 5, characterised in that an albumin or a haemocyanine is used as carrier peptide.

7. Use of the fibrin-specific antibody of claim 1 for the selective qualitative and/or quantitative determination of fibrin.

8. Use of the fibrin-specific antibody according to claim 1 for the selective determination of fibrin according to the principle of the immunoassay by incubation with at least two different receptors, of which the first receptor R₁ is present in solid phase and is bindable with the fibrin or a complex of fibrin and a receptor and at least one further receptor R₂ which is bindable with fibrin or a complex of fibrin and a receptor is present dissolved in liquid phase and whereby one receptor R₂ carries a labelling, separation of the solid from the liquid phase and measurement of the labelling in one of the phases, characterised in that, as one of the receptors, one uses an antibody which is specifically bindable with the peptide sequence Leu-Ile-Asp-Gly-Lys-Met and discriminates between fibrin and fibrinogen.

9. Use according to claim 8, characterised in that, as antibody bound to the solid phase, one uses an antibody which is specifically bindable with the peptide sequence Leu-Ile-Asp-Gly-Lys-Met and, as soluble receptor, uses another antibody bindable with fibrin which carries a labelling.

10. Use according to claim 8, characterised in that, as receptor bound to the solid phase, one uses a receptor bindable with fibrin and, as soluble receptor, uses an antibody which is specifically bindable with the peptide sequence Leu-Ile-Asp-Gly-Lys-Met and carries a labelling.

11. Use according to one of claims 8 to 10, characterised in that the labelling is a radio-active compound, an enzyme, a chemiluminescent or fluorescing compound.

12. Use according to one of claims 8 to 11, characterised in that the antibody which is specifically bindable with the peptide sequence Leu-Ile-Asp-Gly-Lys-Met is a monoclonal antibody.

13. Use of the fibrin-specific antibody according to claim 1 for the qualitative and quantitative determination in vitro of fibrin and fibrin coagula in the human blood circulation and tissues.

14. Use of the fibrin-specific antibody according to claim 1 for the determination of thrombi in blood vessels.

15. Fibrin-specific antibody according to claim 1 for the production of a medicament for the specific dissolving of thrombi, characterised in that one couples a lysing substance on to the fibrin-specific antibody.

16. Reagent for the selective quantitative determination of fibrin, containing a receptor R₁ bound to a solid phase which is bindable with fibrin or a complex of fibrin and a receptor and at least one receptor R₂ present in soluble phase which is bindable with fibrin or a complex of fibrin and a receptor and whereby a soluble receptor R₂ carries a labelling, characterised in that one of the receptors is an antibody which is specifically bindable with the peptide sequence Leu-Ile-Asp-Gly-Lys-Met and discriminates between fibrin and fibrinogen.

## Claims (Claims for the following Contracting State(s) : ES)

1. Process for the obtaining of a monoclonal antibody which is specifically bindable with the peptide sequence Leu-Ile-Asp-Gly-Lys-Met and discriminates between fibrin and fibrinogen, characterised in that one immunises mice with a peptide which carries the amino acid sequence Leu-Ile-Asp-Gly-Lys-Met, fusions B-lymphocytes from the spleen of the immunised animals with myeloma cells, clones the hybridoma cells formed, isolates the clones bindable with the sequence Leu-Ile-Asp-Gly-Lys-Met, selections for discrimination between fibrin and fibrinogen and cultures and recovers the monoclonal antibodies formed by them.

2. Process according to claim 1, characterised in that one uses myeloma cells which themselves produce no immune globulin.

3. Process according to claim 1 or 2, characterised in that, as immunogen, one uses a conjugate which consists of a carrier peptide to which is bound, via a spacer, a peptide with 6 to 20 amino acids and which contains the sequence Leu-Ile-Asp-Gly-Lys-Met.

4. Process according to claim 3, characterised in that, as immunogen, one uses a conjugate in which a peptide with the sequence Leu-Ile-Asp-Gly-Lys-Met is bound to a carrier peptide via a spacer.

5. Process according to claim 3 or 4, characterised in that an albumin or a haemocyanine is used as carrier peptide.

6. Use of the fibrin-specific antibody produced according to claim 1 for the selective qualitative and/or quantitative determination of fibrin.

7. Use of the fibrin-specific antibody produced according to claim 1 for the selective determination of fibrin according to the principle of the immunoassay by incubation with at least two different receptors, of which the first receptor R₁ is present in solid phase and is bindable with fibrin or a complex of fibrin and a receptor and at least one further receptor R₂ which is bindable with fibrin or a complex of fibrin and a receptor is present dissolved in liquid phase and whereby one receptor R₂ carries a labelling, separation of the solid from the liquid phase and measurement of the labelling in one of the phases, characterised in that, as one of the receptors, one uses an antibody which is specifically bindable with the peptide sequence Leu-Ile-Asp-Gly-Lys-Met and discriminates between fibrin and fibrinogen.

8. Use according to claim 7, characterised in that, as antibody bound to the solid phase, one uses an antibody which is specifically bindable with the peptide sequence Leu-Ile-Asp-Gly-Lys-Met and, as soluble receptor, uses another antibody bindable with fibrin which carries a labelling.

9. Use according to claim 7, characterised in that, as receptor bound to the solid phase, one uses a receptor bindable with fibrin and, as soluble receptor, uses an antibody which is specifically bindable with the peptide sequence Leu-Ile-Asp-Gly-Lys-Met and carries a labelling.

10. Use according to one of claims 7 to 9, characterised in that the labelling is a radio-active compound, an enzyme, a chemiluminescent or fluorescing compound.

11. Use according to one of claims 7 to 10, characterised in that the antibody which is specifically bindable with the peptide sequence Leu-Ile-Asp-Gly-Lys-Met is a monoclonal antibody.

12. Use of the antibody produced according to claim 1 for the qualitative and quantitative determination in vitro of fibrin and fibrin coagula in human blood circulation and tissues.

13. Use of the fibrin-specific antibody produced according to claim 1 for the determination of thrombi in blood vessels.

14. Reagent for the selective quantitative determination of fibrin, containing a receptor R₁ bound to a solid phase which is bindable with fibrin or a complex of fibrin and a receptor and at least one receptor R₂ present in soluble phase which is bindable with fibrin or a complex of fibrin and a receptor and whereby a soluble receptor R₂ carries a labelling, characterised in that one of the receptors is an antibody which is specifically bindable with the peptide sequence Leu-Ile-Asp-Gly-Lys-Met and discriminates between fibrin and fibrinogen.

## Revendications (Revendications pour l'(les) Etat(s) contractant(s) suivant(s) : suivants : AT, BE, CH, DE, FR, GB, GR, IT, LI, LU, NL, SE)

1. Anticorps spécifique de la fibrine, capable de se lier spécifiquement à la séquence peptidique Leu-lle-Asp-Gly-Lys-Met et faisant une discrimination entre la fibrine et le fibrinogène.

2. Procédé d'obtention d'un anticorps monoclonal qui est capable de se lier spécifiquement à la séquence peptidique Leu-lle-Asp-Gly-Lys-Met et qui fait une discrimination entre la fibrine et le fibrinogène, caractérisé en ce que l'on immunise des souris avec un peptide qui porte la séquence Leu-lle-Asp-Gly-Lys-Met, on fusionne des lymphocytes B provenant de la rate des animaux immunisés avec des cellules de myélome, on clone les cellules d'hybridome formées, on isole les clones qui sont capables de se lier spécifiquement à la séquence Leu-Ile-Asp-Gly-Lys-Met, on les sélectionne sur la base de la discrimination entre la fibrine et le fibrinogène et on les cultive, et on obtient l'anticorps monoclonal formé par eux.

3. Procédé selon la revendication 2, caractérisé en ce que l'on utilise des cellules de myélome qui, elles-mêmes, ne produisent pas d'immunoglobuline.

4. Procédé selon la revendication 2 ou 3, caractérisé en ce que l'on utilise comme immunogène un conjugué qui consiste en un peptide-support auquel est lié par l'intermédiaire d'un es- paceur un peptide de 6 à 20 acides aminés qui contient la séquence Leu-lle-Asp-Gly-Lys-Met.

5. Procédé selon la revendication 4, caractérisé en ce que l'on utilise comme immunogène un conjugué dans lequel un peptide contenant la séquence Leu-lle-Asp-Gly-Lys-Met est lié à un peptide-support par l'intermédiaire d'un espa- ceur.

6. Procédé selon la revendication 4 ou 5, caractérisé en ce que l'on utilise l'albumine ou une hémocyanine comme peptide-support.

7. Utilisation de l'anticorps spécifique de la fibrine selon la revendication 1 pour la détermination sélective qualitative et/ou quantitative de la fibrine.

8. Utilisation de l'anticorps spécifique de la fibrine selon la revendication 1 pour la détermination sélective de la fibrine selon le principe de l'immuno-analyse par incubation avec au moins deux récepteurs différents parmi lesquels le premier récepteur R₁ se trouve en phase solide et est capable de se lier à la fibrine ou à un complexe constitué par la fibrine et par un récepteur, et au moins un autre récepteur R₂, qui est capable de se lier à la fibrine ou à un complexe constitué par la fibrine et par un récepteur, est dissous en phase liquide et où un récepteur R₂ porte un marqueur, séparation de la phase solide et de la phase liquide et mesure du marqueur dans l'une des phases, caractérisée en ce que l'on utilise pour l'un des récepteurs un anticorps qui est capable de se lier spécifiquement à la séquence peptidique Leu-lle-Asp-Gly-Lys-Met et qui fait une discrimination entre la fibrine et le fibrinogène.

9. Utilisation selon la revendication 8, caractérisée en ce que l'on utilise comme anticorps lié à la phase solide un anticorps qui est capable de se lier spécifiquement à la séquence peptidique Leu-lle-Asp-Gly-Lys-Met et comme récepteur soluble un autre anticorps capable de se lier à la fibrine et qui porte un marqueur.

10. Utilisation selon la revendication 8, caractérisée en ce que l'on utilise comme récepteur lié à la phase solide un récepteur capable de se lier à la fibrine et comme récepteur soluble un anticorps qui est capable de se lier spécifiquement à la séquence peptidique Leu-Ile-Asp-Gly-Lys-Met et qui porte un marqueur.

11. Utilisation selon l'une des revendications 8 à 10, caractérisée en ce que le marqueur est un composé radioactif, une enzyme ou un composé chimiluminescent ou fluorescent.

12. Utilisation selon l'une des revendications 8 à 11, caractérisée en ce que l'anticorps qui est capable de se lier spécifiquement à la séquence peptidique Leu-lle-Asp-Gly-Lys-Met est un anticorps monoclonal.

13. Utilisation de l'anticorps spécifique de la fibrine selon la revendication 1 pour la détermination qualitative et quantitative in vitro de la fibrine et des caillots de fibrine dans la circulation sanguine humaine et les tissus humains.

14. Utilisation de l'anticorps spécifique de la fibrine selon la revendication 1 pour la mise en évidence de thrombus dans les vaisseaux sanguins.

15. Anticorps spécifique de la fibrine selon la revendication 1 pour la préparation d'un médicament pour la dissolution spécifique des thrombus, caractérisé en ce que l'on couple une substance lytique à l'anticorps spécifique de la fibrine.

16. Réactif pour la détermination quantitative sélective de la fibrine, contenant un récepteur R₁ lié à une phase solide, qui est capable de se lier à la fibrine ou à un complexe constitué par la fibrine et par un récepteur, et au moins un récepteur R₂ présent en phase soluble qui est capable de se lier à la fibrine ou à un complexe constitué par la fibrine et par un récepteur et où un récepteur soluble R₂ porte un marqueur, caractérisé en ce que l'un des récepteurs est un anticorps qui est capable de se lier spécifiquement à la séquence peptidique Leu-lle-Asp-Gly-Lys-Met et qui fait une discrimination entre la fibrine et le fibrinogène.

## Revendications (Revendications pour l'(les) Etat(s) contractant(s) suivant(s) : suivant : ES)

1. Procédé d'obtention d'un anticorps monoclonal qui est capable de se lier spécifiquement à la séquence peptidique Leu-lle-Asp-Gly-Lys-Met et qui fait une discrimination entre la fibrine et le fibrinogène, caractérisé en ce que l'on immunise des souris avec un peptide qui porte la séquence Leu-lle-Asp-Gly-Lys-Met, on fusionne des lymphocytes B provenant de la rate des animaux immunisés avec des cellules de myélome, on clone les cellules d'hybridome formées, on isole les clones qui sont capables de se lier spécifiquement à la séquence Leu-Ile-Asp-Gly-Lys-Met, on les sélectionne sur la base de la discrimination entre la fibrine et le fibrinogène et on les cultive, et on obtient l'anticorps monoclonal formé par eux.

2. Procédé selon la revendication 1, caractérisé en ce que l'on utilise des cellules de myélome qui, elles-mêmes, ne produisent pas d'immunoglobuline.

3. Procédé selon la revendication 1 ou 2, caractérisé en ce que l'on utilise comme immunogène un conjugué qui consiste en un peptide-support auquel est lié par l'intermédiaire d'un es- paceur un peptide de 6 à 20 acides aminés qui contient la séquence Leu-lle-Asp-Gly-Lys-Met.

4. Procédé selon la revendication 3, caractérisé en ce que l'on utilise comme immunogène un conjugué dans lequel un peptide contenant la séquence Leu-lle-Asp-Gly-Lys-Met est lié à un peptide-support par l'intermédiaire d'un espa- ceur.

5. Procédé selon la revendication 3 ou 4, caractérisé en ce que l'on utilise l'albumine ou une hémocyanine comme peptide-support.

6. Utilisation de l'anticorps spécifique de la fibrine préparé selon la revendication 1 pour la détermination sélective qualitative et/ou quantitative de la fibrine.

7. Utilisation de l'anticorps spécifique de la fibrine préparé selon la revendication 1 pour la détermination sélective de la fibrine selon le principe de l'immunoanalyse par incubation avec au moins deux récepteurs différents parmi lesquels le premier récepteur R₁ se trouve en phase solide et est capable de se lier à la fibrine ou à un complexe constitué par la fibrine et par un récepteur, et au moins un autre récepteur R₂, qui est capable de se lier à la fibrine ou à un complexe constitué par la fibrine et par un récepteur, est dissous en phase liquide et où un récepteur R₂ porte un marqueur, séparation de la phase solide et de la phase liquide et mesure du marqueur dans l'une des phases, caractérisée en ce que l'on utilise pour l'un des récepteurs un anticorps qui est capable de se lier spécifiquement à la séquence peptidique Leu-lle-Asp-Gly-Lys-Met et qui fait une discrimination entre la fibrine et le fibrinogène.

8. Utilisation selon la revendication 7, caractérisée en ce que l'on utilise comme anticorps lié à la phase solide un anticorps qui est capable de se lier spécifiquement à la séquence peptidique Leu-lle-Asp-Gly-Lys-Met et comme récepteur soluble un autre anticorps capable de se lier à la fibrine et qui porte un marqueur.

9. Utilisation selon la revendication 7, caractérisée en ce que l'on utilise comme récepteur lié à la phase solide un récepteur capable de se lier à la fibrine et comme récepteur soluble un anticorps qui est capable de se lier spécifiquement à la séquence peptidique Leu-Ile-Asp-Gly-Lys-Met et qui porte un marqueur.

10. Utilisation selon l'une des revendications 7 à 9, caractérisée en ce que le marqueur est un composé radioactif, une enzyme ou un composé chimiluminescent ou fluorescent.

11. Utilisation selon l'une des revendications 7 à 10, caractérisée en ce que l'anticorps qui est capable de se lier spécifiquement à la séquence peptidique Leu-lle-Asp-Gly-Lys-Met est un anticorps monoclonal.

12. Utilisation de l'anticorps spécifique de la fibrine préparé selon la revendication 1 pour la détermination qualitative et quantitative in vitro de la fibrine et des caillots de fibrine dans la circulation sanguine humaine et les tissus humains.

13. Utilisation de l'anticorps spécifique de la fibrine préparé selon la revendication 1 pour la mise en évidence de thrombus dans les vaisseaux sanguins.

14. Réactif pour la détermination quantitative sélective de la fibrine, contenant un récepteur R₁ lié à une phase solide, qui est capable de se lier à la fibrine ou à un complexe constitué par la fibrine et par un récepteur, et au moins un récepteur R₂ présent en phase soluble qui est capable de se lier à la fibrine ou à un complexe constitué par la fibrine et par un récepteur et où un récepteur soluble R₂ porte un marqueur, caractérisé en ce que l'un des récepteurs est un anticorps qui est capable de se lier spécifiquement à la séquence peptidique Leu-lle-Asp-Gly-Lys-Met et qui fait une discrimination entre la fibrine et le fibrinogène.
